# EUROPEAN PATENT APPLICATION

(11) **EP 4 202 941 A1**
(43) Date of publication of application: **28.06.2023**
(21) Application number: 21306894.3
(22) Date of filing: 21.12.2021
(51) Int. Cl.: G16B 5/30, G06N 20/00, G16B 25/10, G16B 40/20

(54) **INFERRENCE OF A GENE EXPRESSION PROFILE**

(71) Applicant: Dassault Systèmes, 78140 Vélizy-Villacoublay (FR)
(72) Inventor: Séchet, Pauline, Biot (FR); Ball, Arthur, Vélizy-Villacoublay (FR)
(74) Representative: Bandpay & Greuter

(57) **Abstract**

The invention notably relates to a computer-implemented method for training a neural network for inferring a gene expression profile. The method comprises obtaining a matrix of potential regulations between genes of a set of genes of a sequence of reference genome, obtaining a neural network having an input layer of nodes and an output layer of nodes, the input layer and the output layer having an equivalent node for representing each gene of the set of genes of the sequence of the reference genome, each node of the input layer representing a regulator gene and each node of the output layer representing a regulated gene, adding connections to the neural network from the nodes of the input layer to the nodes of the output layer, the added connections being extracted from the obtained matrix of potential regulations, training the neural network by using a set of gene expression profiles of the observed biological process, each connection of the trained the neural network being weighted, and removing connections of the trained neural network having an insignificant weight value.

## Description

### FIELD OF THE INVENTION

The disclosure relates to the field of data science applied in biology, and more specifically to methods, data structures and systems related to inferring a gene expression profile.

### BACKGROUND

Data science is gaining wide importance in the field of biology. Data regarding biological process are increasingly available and can be used to infer biological processes more and more accurately. Especially, data science can be applied to any biological process implicating gene expression modification assuming that wanted data are provided.

In this context, several approaches are foreseeable to reconstruct a transcriptional regulatory network. A gene regulatory network (GRN) is a collection of molecular regulators that interact with each other and with other substances in the cell to govern the gene expression levels of mRNAand proteins which, in turn, determine the function of the cell.

A first approach is called logicTRN and has been discussed in Bin Yan, Daogang Guan, Chao Wang, Junwen Wang, Bing He, Jing Qin, Kenneth R Boheler, Aiping Lu, Ge Zhang, and Hailong Zhu. An integrative method to decode regulatory logics in gene transcription. Nature communications, 8(1):1044, 2017. This first approach uses TF-DNA-binding information (through chromatin data) and gene expression data to decipher TF regulatory logics in gene transcription. For a given target gene (TG), a set of TF are assumed to be able to bind to its promoter. Observations and differential equations model the different regulatory logics of a gene and extending this technique to all genes allows a global vision of regulatory mechanisms. This approach is interesting but relies on a boolean simulation, while a quantitative simulation may be preferable.

The second approach first predicts transcription factor (TF) bindings at each stage at any position on the genome using a Logistic Regression model and then reconnects the different time points using a Time Varying Dynamic Bayesian Network. Around hundred features are extracted from the data to build the model.

The main drawback of this approach is that it builds a stage-specific rules. It can therefore be seen as a simple observation and not a dynamic network; there is not enough abstraction for the model to be interesting.

The third approach relies on Recurrent Neural Networks (RNN) as described in Abhinandan Khan, Sudip Mandal, Rajat Kumar Pal, and Goutam Saha. Construction of gene regulatory networks using recurrent neural networks and swarm intelligence. Scientifica, 2016. The regulation of the expression of any particular gene, by another gene or a group of genes, can be expressed by Recurrent Neural Networks (RNN). RNNs are connectionist models that capture the dynamics of sequences via cycles in a sequence of nodes. A RNN is composed individual units (neurons) connected to each other, interacting non-linearly and for which it exists at least a cycle in the structure. Neurons are connected by weighted edges. The output of a neuron is a non-linear combination of its inputs. In their unfolded form, RNNs are comparable to classical artificial neural networks with equality constraints on the network's weights. However, this third approach suffers the drawback that the networks is built for known regulations in the literature. This means that the model is built with *a priori* (e.g with using literature knowledge). The problem of *a priori* is that the *a priori* is not exhaustive and the current comprehension of regulating mechanisms is not totally satisfying. The inferences are refrained to the current knowledges.

To sum up, the current approaches are purely descriptive and merely describe the mechanisms of regulation, which gene regulates which other gene. This is not satisfactory as these approaches do not allow to be predictive over time, and therefore do not allow to predict the future expression of a gene given a state of a gene expression.

Within this context, there is still a need for an improved method for inferring a gene expression profile.

### SUMMARY OF THE INVENTION

It is therefore provided a computer-implemented method for training a neural network for inferring a gene expression profile. The method comprises:
- obtaining a matrix of potential regulations between genes of a set of genes of a sequence of reference genome, the matrix of potential regulations describing connections between regulator genes and regulated genes, a regulator gene encoding at least one transcription factor regulating at least one regulated gene, a connection representing at least one observed regulation of the regulated gene by the regulator gene in at least one time series of an observed biological process involving the genes of the set of genes of the sequence of the reference genome;
- obtaining a neural network having an input layer of nodes and an output layer of nodes, the input layer and the output layer having an equivalent node for representing each gene of the set of genes of the sequence of the reference genome, each node of the input layer representing a regulator gene and each node of the output layer representing a regulated gene;
- adding connections to the neural network from the nodes of the input layer to the nodes of the output layer, the added connections being extracted from the obtained matrix of potential regulations;
- training the neural network by using a set of gene expression profiles of the observed biological process, each connection of the trained the neural network being weighted; and
- removing connections of the trained neural network having an insignificant weight value.

The method may comprise one or more of the following:
- the removing the connections of the trained neural network having an insignificant weight comprises performing for each connection of the trained neural network: - obtaining a value of a threshold of insignificance representing a modification of an expression of the regulated gene in a range of an experimental error; - removing the connection to the regulated gene if the weight value is smaller than the threshold of insignificance;
- the obtained matrix of potential regulations between genes of a set of genes of a sequence of reference genome has been computed by: - identifying, for each gene of the set of genes of the sequence of the reference genome, one or more transcription factor binding sites and the respective transcription factor(s) bound on the one or more transcription factor binding sites; - for each identified bound transcription factor: -- identifying one or more potentially regulated genes; -- identifying a potentially regulator gene encoding the bound transcription factor; and -- connecting the regulator gene and the one or more regulated genes.

- the identifying one or more potentially regulated genes comprises: - determining, from a gene location map of the genes of the set of genes of the sequence of the reference genome, if one or more genes are in the frame of a predetermined number of base pairs around the identified bound transcription factor; and - identifying the one or more genes are in the frame of a predetermined number of base pairs around the identified bound transcription factor as potentially regulated genes.
- the predetermined number of base pairs is smaller than 15000, preferably smaller than 10000.
- the identifying, for each gene of the set of genes of the sequence of the reference genome, one or more transcription factor binding sites comprises: - performing a peak calling operation on chromatin accessibility data of the set of genes of the sequence of the reference genome, thereby identifying peaks; - identifying one or more hollows for each identified peak, thereby obtaining footprints of a past presence of transcription factor on the chromatin accessibility data of the set of genes of the sequence of the reference genome; - comparing the obtained footprints to motifs of known transcription factors; and - identifying, as a result of the comparing, which transcriptions factor has been bound to each footprint.
- the obtained matrix of potential regulations between genes of a set of genes of a sequence of reference genome has been computed by: - obtaining a matrix of potential regulations for each time series of the observed biological process, thereby obtaining a set of matrices of potential regulations; and - merging the matrix of potential regulations of the set of matrices of potential regulations;
- a connection described for each time series of the observed biological process is equivalent to a connection described for one of the time series of the observed biological process.

It is further provided a computer-implemented method of use of the above trained neural network for inferring a gene expression profile. The method of use comprises:
- providing input data including a gene expression for a time series of the observed biological process involving the genes of the set of genes of the sequence of the reference genome;
- applying the trained neural network to the input data to infer a future gene expression.

It is further provided a computer-implemented method for obtaining a matrix of potential regulations according to the above method. The method may comprise obtaining experimental data and extracting from the experimental data the matrix of potential regulations between genes of a set of genes of a sequence of reference genome, the matrix of potential regulations describing connections between regulator genes and regulated genes, a regulator gene encoding at least one transcription factor regulating at least one regulated gene, a connection representing at least one observed regulation of the regulated gene by the regulator gene in at least one time series of an observed biological process involving the genes of the set of genes of the sequence of the reference genome.

The method for obtaining a matrix of potential regulations may further comprise one or more of the following:
- the obtained matrix of potential regulations between genes of a set of genes of a sequence of reference genome is computed by: - identifying, for each gene of the set of genes of the sequence of the reference genome, one or more transcription factor binding sites and the respective transcription factor(s) bound on the one or more transcription factor binding sites; - for each identified bound transcription factor:identifying one or more potentially regulated genes; -- identifying a potentially regulator gene encoding the bound transcription factor; and -- connecting the regulator gene and the one or more regulated genes.
- the identifying one or more potentially regulated genes comprises: - determining, from a gene location map of the genes of the set of genes of the sequence of the reference genome, if one or more genes are in the frame of a predetermined number of base pairs around the identified bound transcription factor; and - identifying the one or more genes are in the frame of a predetermined number of base pairs around the identified bound transcription factor as potentially regulated genes.
- the predetermined number of base pairs is smaller than 15000, preferably smaller than 10000.
- the identifying, for each gene of the set of genes of the sequence of the reference genome, one or more transcription factor binding sites comprises: - performing a peak calling operation on chromatin accessibility data of the set of genes of the sequence of the reference genome, thereby identifying peaks; - identifying one or more hollows for each identified peak, thereby obtaining footprints of a past presence of transcription factor on the chromatin accessibility data of the set of genes of the sequence of the reference genome; - comparing the obtained footprints to motifs of known transcription factors; and - identifying, as a result of the comparing, which transcriptions factor has been bound to each footprint.
- the obtained matrix of potential regulations between genes of a set of genes of a sequence of reference genome has been computed by: - obtaining a matrix of potential regulations for each time series of the observed biological process, thereby obtaining a set of matrices of potential regulations; and - merging the matrix of potential regulations of the set of matrices of potential regulations;
- a connection described for each time series of the observed biological process is equivalent to a connection described for one of the time series of the observed biological process;

It is further provided a data structure comprising a trained neural network according the above method, and/or a dataset formed according to the method for obtaining the matrix of potential regulations, and/or a computer program comprising instructions for performing the above method, the above method of use, and/or the method for obtaining the matrix of potential regulations.

It is further provided a computer readable storage medium having recorded thereon the data structure.

It t is further provided a device comprising a data storage medium having recorded thereon the data structure.

### BRIEF DESCRIPTION OF THE DRAWINGS

Embodiments of the invention will now be described, by way of non-limiting example, and in reference to the accompanying drawings, where:
- FIG. 1 shows an example of the system
- FIG. 2 shows a flowchart of an example of the method;
- FIG. 3 shows an example of gene expression;
- FIG. 4 shows examples of transcription factor binding motifs;
- FIG. 5 illustrates the principle of a matrix of potential regulation;
- FIG. 6 illustrates a matrix of potential regulation; and
- FIG. 7 shows an example of the system.

### DETAILED DESCRIPTION OF THE INVENTION

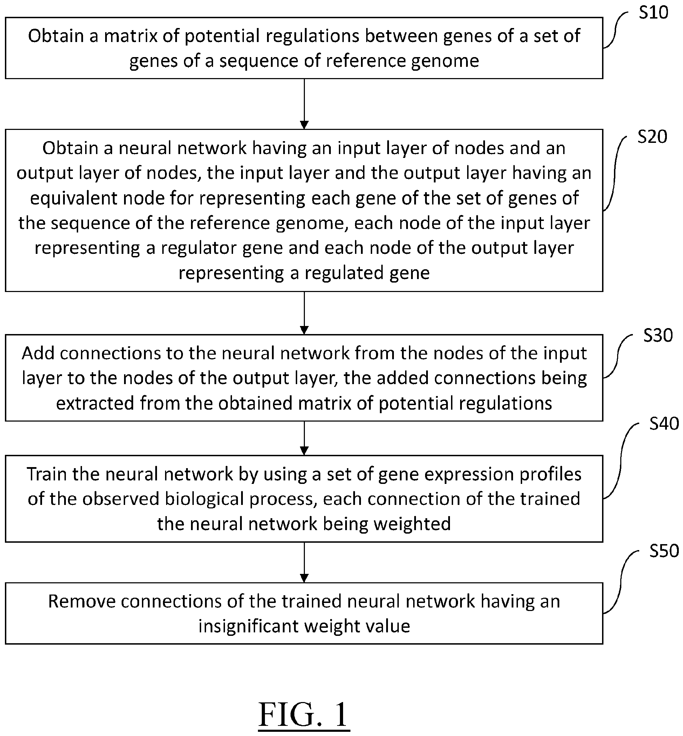

With reference to the flowchart of FIG. 1, it is proposed a computer-implemented method for training a neural network for inferring a gene expression profile. A gene expression profile identifies all of the genes in a cell or tissue that are making messenger RNA. A gene expression profile can be inferred to find and/or diagnose a disease and/or condition and/or to see how well a body responds to treatment. The method comprises obtaining a matrix of potential regulations between genes of a set of genes of a sequence of reference genome. The matrix of potential regulations describes connections between regulator genes and regulated genes. A regulator gene is a gene encoding at least one transcription factor (noted TF). A regulated gene is a gene whose expression is controlled by the regulator gene. A connection between a regulator gene and a regulated gene represents at least one observed regulation of the regulated gene by the regulator gene. The observation of the regulation has been made in at least one time series of an observed biological process involving the genes of the set of genes of the sequence of the reference genome. The method further comprises obtaining a neural network. The neural network has an input layer of nodes and an output layer of nodes. The input layer and the output layer have an equivalent node for representing each gene of the set of genes of the sequence of the reference genome. Each node of the input layer represents a regulator gene and each node of the output layer representing a regulated gene. The method also comprises adding connections to the neural network from the nodes of the input layer to the nodes of the output layer. The added connections are extracted from the matrix of potential regulations that has been obtained. Next, the method comprises training the neural network with added connections by using a set of gene expression profiles of the observed biological process. Each connection of the trained the neural network is weighted. Then, the method comprises removing connection(s) of the trained neural network with an insignificant weight value.

The methods presented herein are all part of a global solution for performing an inference of a gene expression profile. The dataset-forming method allows obtaining a dataset that can be used in learning method. The learning method allows to train a neural network that can be used the use method. In turn, the use method allows to perform an inference of a gene expression profile.

The proposed solution allows to perform an inference of a gene expression profile without an a priori, that is without using literature knowledge.

The dataset provides a matrix of potential regulations between genes of a set of genes of a sequence of reference genome. The dataset can thus serve to build and train a neural network adapted in the use method to perform an inference of a gene expression profile, that is, to predict gene expression in the future given a gene expression profile. It can also be used to predict gene expression in the future given a perturbed gene expression profile. The data used to learn the neural network are gene expression time series : the expression of every gene at different time points (the same for all genes).Once trained it uses gene expression profile as input and gives gene expression profile as output.

The methods uses data to find possible regulations and give this information to the neural network as a starting point. Instead of looking in the knowledge for any potential known regulation, potential regulations between genes are built. Building the model frees the invention from the classical knowledge based model creation. Instead of a fully connected non-informed brute force neural network, an informed structure is built. The neural network structure is built with information learned from data. Each connection in the network represents a possible regulation deduced, e.g. by chromatin accessibility data. The problem of a priori is solved as data is to create the network instead of the literature. The method also provides a learning process, wherein each node of the network represents a gene. The present invention creates a model with as many genes as is given in input (number of gene expressions). Any gene considered in the data can be added to the model. The construction of the model is incremental, that is to say that genes are added as they are found in the learning data. The training of the networks "learns" regulations between genes. Only potential regulations previously defined are learned. The weight of the edges between nodes defines the strength of the regulation.

The invention is thus independent of a biological context. This allows to perform inference on cellular senescence process, being understood that any biological process car be used by the methods as long as required data is given as an input to the model. Hence, the invention uses data instead of knowledge to build the structure and therefore is able to cover/discover unknown gene regulations. This is particularly important as gene regulations are highly dependent on the biological context. The invention can predict future gene expression given an expression profile. The invention can also predict a future gene expression given modified gene expression profile; Thus the invention can be used to simulate what happens if some gene expressions are modified. The invention provides hints about potential unknown (in the literature) regulations between genes looking at the trained model; therefore, even if a regulation has not been observed, the invention provides hints that the unknown regulation is possible.

The methods are computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory, the memory having recorded thereon a computer program comprising instructions for performing the method. The memory may also store a database. The memory is any hardware adapted for such storage, possibly comprising several physical distinct parts (e.g., one for the program, and possibly one for the database).

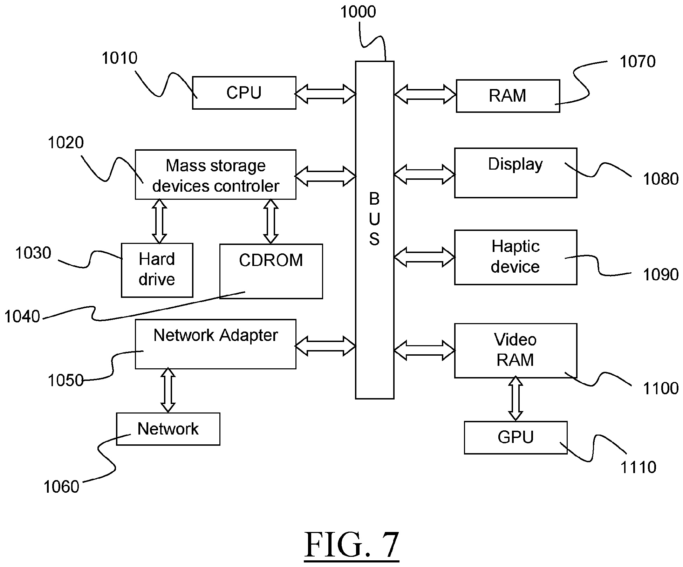

FIG. 7 shows an example of the system, wherein the system is a client computer system.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks; and CD-ROM disks 1040. Any of the foregoing may be supplemented by, or incorporated in, specially designed ASICs (application-specific integrated circuits). A network adapter 1050 manages accesses to a network 1060, for example so as to access remote data (the dataset may indeed be stored on local memory such as hard drive 1030, and/or on distant memory such as a cloud). The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method.

The trained neural network is configured for inferring a gene expression profile. In other words, the trained neural network is configured to gives gene expression profile as output by taking as input gene expression profile.

The flowchart of FIG. 1 is now discussed.

At **S10**, a matrix of potential regulations is obtained. The matrix of potential regulations describes potential regulations between genes of a set of genes of a sequence of reference genome. The reference genome may be any genome, e.g. reference genome is the human genome. A sequence of reference genome means that all or part of the reference genome is supported by the matrix potential regulation. The sequence of reference genome thus comprises a set of genes, and the matrix of potential regulations concerns the genes of the set of genes.

The matrix of potential regulations describes connections between regulator genes and regulated genes. A regulator gene is a gene involved in controlling the expression of one or more other genes of the set of genes. A regulated gene (of the set of genes) is a gene whose expression is controlled by one or more regulated genes.

Gene expression is the process by which information from a gene is used in the synthesis of a functional gene product that enables the gene to produce end products, protein or non-coding RNA; gene expression ultimately affect a phenotype, as the final effect. Gene expression is a well-known process in biology and will be not discussed in the present disclosure.

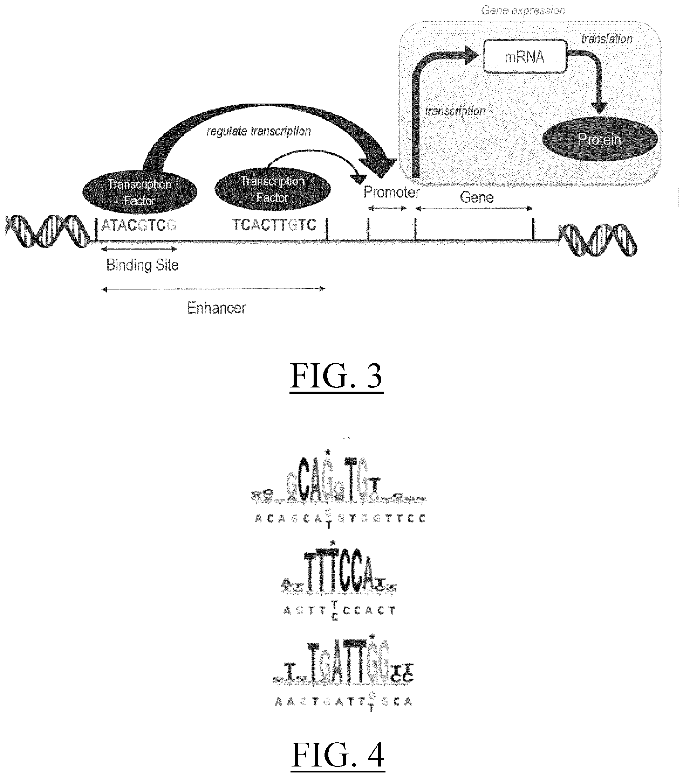

For the sake of illustration only, the principles of gene expression in Eucaryote cell are shown on FIG. 3. The DNA sequence of a gene is transcribed to make an mRNA molecule, and the mRNA molecule is then translated in the end product (a protein in FIG. 3). Transcription is performed in the nucleus by three types of mRNA polymerases, each of which needs a special DNA sequence called the promoter and a set of DNA-binding proteins-transcription factors-to initiate the process (see regulation of transcription below). Transcription Factors are proteins regulating genes. The human genome has around 700 different Transcription Factors that bind close (in a frame of 10 000bp) to a gene to regulate it. Each transcription has a preferred DNA "motif" to bind with. Like a puzzle piece it will bind or not depending on the motif. The genes are often flanked by several transcription factor binding sites (TFBS) for distinct transcription factors, and efficient expression of each of these genes requires the cooperative action of several different transcription factors.

A regulator gene encodes at least one transcription factor (TF) that regulates at least one regulated gene. A connection between a regulator gene and a regulated gene represents at least one observed regulation of the regulated gene by the regulator gene. A connection therefore represents a transcription factor (TF) that is encoded by a regular gene and that regulates one or more genes. It is to be understood that a gene is regulated by at least one regular gene, and that a regular gene may regulate one or several regulated genes.

The observation of connection between a regulator gene and a regulated gene is made for at least one time series of an observed biological process involving the genes of the set of genes of the sequence of the reference genome. A biological process is a process that is vital for an organism to live. A biological process comprises many chemical reactions and involves gene expressions. For example, cellular senescence process is a biological process. It is to be understood that the present invention can be used for any biological process that comprises at least one gene expression.

Genes coding for gene products are generally regulated in a coordinated manner in the course of a biological process. Therefore, the roles of a gene and gene expression evolves throughout the time course of the biological process. A biological process is thus (experimentally) observed throughout one or time series. The time series may be of an identical length, or not.

As seen hereinabove, the expression level of a gene depends on its expression at the previous timepoint for an observed biological process, and the expression level of a gene also depends on Transcription Factor (TF). Hence, the expression level of a gene depends on Transcription Factor (TF) bindings at the previous timepoint ; the matrix of potential regulations that is obtained at S10 represents the expression level of genes for at least one time point of an observed biological process.

The matrix of potential regulations (the dataset) is obtained (S10). By obtaining it is meant the matrix of potential regulations is a dataset provided to (already built) and used by the method of training the neural network. Alternatively, the matrix of potential regulation is built by and used by the method. In other words, the dataset - obtained for training the neural network- can be built online (that is, in the course of the training) or offline (that is, at an earlier stage prior the training).

Examples of the obtaining S10 are now discussed. These examples can indifferently apply to the online or offline modes. In these examples, an identification is carried out, for each gene of the sequence of the reference genome, of one or more transcription factor binding sites. The respective transcription factors bound with the one or more transcription factor binding sites are identified too. As discussed in reference to FIG. 3, the genes are flanked by several transcription factor binding sites (TFBS) for distinct transcription factors (TF). Thus the identification of a TBFS allows identifying one or more TF that are potentially bound to the TBFS.

In examples, the TBFS and their TF may be identified by analyzing the chromatin accessibility data of the set of genes of the sequence of the reference genome. Chromatin is a complex of DNA and protein found in eukaryotic cells. The primary function of Chromatin is packaging long DNA molecules into more compact, denser structures that fit the cell nucleus. Changes in Chromatin structure are associated with DNA replication and gene expression.

In an example, the analysis of the chromatin accessibility data for identifying TBFS and theirTF may be carried out by performing the following three steps, namely (i) a peal calling, (ii) a foot printing, and (iii) a motif matching. These three steps allow to detect the phenomenon discussed in reference to FIG. 3: transcription factors bind to DNA to regulate transcription and in the end gene expression. The following steps (i), (ii) and (iii) are an example of implementation for extracting information from chromatin accessibility data (for example DNAse-seq or ATAC-seq) to understand which genes may regulate which other genes in the studied biological process.
(i) A peak calling operation is performed on chromatin accessibility data of the set of genes of the sequence of the reference genome, thereby identifying peaks on chromatin.

Peak calling operation allows to find a "large area" of the genome where any transcription factor could bind. Depending on its shape, the chromatin can give access to transcription factors to bind or not. A potential TF binding means that TF could regulate the closest gene to the binding site. The presence of a potential TF binding is not a sufficient but a necessary condition to gene regulation. The chromatin accessibility data provide the information of large areas open enough to let access to a TF to bind. Chromatin accessibility data analysis (peak calling) is a kind of signal analysis where accessible chromatin is visible as a peak. Thus, the identification of peaks on chromatin with the peak calling operation allows to extract from chromatin TBFS.

The peak calling may be performed with any known solution, e.g. but not limited to, with a tool referred to as Homer. Homer is a software tool for motif discovery and sequencing analysis, as disclosed and discussed in http://homer.ucsd.edu/homer/ngs/peaks.html.

(ii) One or more hollows are identified for each identified peak at (i), thereby obtaining footprints of a past presence of transcription factor on the chromatin accessibility data of the set of genes of the sequence of the reference genome. Foot printing aims at find within the large areas (discovered with the peak calling operation) small areas where any transcription factor (TF) could bind. Looking closer to the peak, there are some hollows within it, indicating the footprint of a transcription factor. Each hollow indicates that a transcription factor was certainly (potentially) bound there at the moment of the observation of the studied biological process, but the sole presence does not provide the information of which TF was bound.

This foot printing step may be performed with any known solution, e.g. but not limited to, with a tool called RGT (Regulatory Genomics Toolbox). RGT is a software tool for analysis of regulatory genomics, as disclosed and discussed in https://www.regulatory-genomics.org/hint/introduction/.

(iii) Then, a motif matching is carried out. The obtained footprints are compared to motifs of known transcription factors. And as a result of the comparing, a transcriptions factor has been bound to each footprint is identified. The motif matching step allows to discover the preferred genome sequence binding motif, and to find which TF bound to the small area identified at (ii). This relies on the fact that a TF has more or less specific genomic sequences it binds to, and this more or less specific genomic sequences is called a motif. Comparing the genomic sequence of the footprint to motifs of all known transcription factors indicates which transcription factor was the most likely bound to a motif.

The motifs are known (e.g. observed in several biological processes) and can be accessed in a public databases. For example, the public database called JASPAR (accessible on http://jaspar.genereg.net/) may be used.

As a result of steps (i), (ii) and (iii), a map of the genome of the observed biological process involving the genes of the set of genes of the sequence of the reference genome is obtained with the position and names of all potentially bound transcription factors.

Referring now to FIG. 4, three examples of transcription factor binding motifs are represented. The size of the letter indicates the probability of preferred nucleotide at that binding position.

Back to the example of the obtaining S10, after the one or more TF that can potentially be bound to the TBFS have been identified, one or more genes that are potentially regulated by each of the said one or more TF are identified.

In examples, the identification of the one or more genes potentially regulated by each of the said one or more transcription factors may rely on a gene location map of the genes of the set of genes of the sequence of the reference genome. The map of locations of genes may be obtained from a library of public maps. For example, maps of location of the genes of the human genome has been described by the Human Genome Project: the Human Genome Project is an international research effort completed in 2003, that determined the sequence of base pairs for each human chromosome. This sequence information allows to provide a more specific address than the cytogenetic location for many genes. A gene's molecular address pinpoints the location of that gene in terms of base pairs, and it describes the gene's precise position on a chromosome and indicates the size of the gene. Knowing the molecular location also allows to determine exactly how far a gene is from other genes on the same chromosome. From the gene location map of the genes of the set of genes of the sequence of the reference genome, one determines if one or more genes are in the frame of a predetermined number of base pairs around the identified bound transcription factor. A bound transcription factor does not regulate every gene on the genome; a bound TF normally regulates genes within a predetermined number of base pairs frame.

In an example, the predetermined number of base pairs may be smaller than 15000, that is comprised between 0 and 15000.

In another example, the predetermined number of base pairs may be smaller than 10000, that is comprised between 0 and 10000.

If one or more genes are present in the frame of a predetermined number of base pairs around an identified bound transcription factor, the transcription factor is considered as potentially regulating that gene. Thus, the one or more genes that are in the frame of the predetermined number of base pairs around the identified bound transcription factor are identified as potentially regulated genes. One understands that it is a potential regulation, and the training of the network (discussed hereinbelow) will help to choose if it is an effective regulation or not.

From now, a matrix of regulation with for each found transcription factor, the list of genes it regulates, is obtained.

A transcription factor can regulate further than the predetermined base pairs depending on the 3D structure of DNA. This possibility may be included with a gene location map of the genes of the set of genes of the sequence of the reference genome that captures this spatial information.

In examples, the matrix of potential regulations between genes of a set of genes of a sequence of reference genome may be obtained for a biological process that is observed for several time series. The biological process is studied through several experiments (possibly under different conditions), and experimental measurements are performed for each experiment. In such an example, a matrix of potential regulations is obtained for each time series of the observed biological process so that a set of matrices of potential regulations is obtained. The matrices of potential regulations (obtained for each time series) are then merged so that one single matrix of potential regulations is obtained. The merge may be carried out so that all information in the set of potential regulation matrices is available in the obtained single matrix of potential regulations.

In examples, the merge may be carried out by performing a one-to-one merge in which the number of observations in the single matrix of potential regulations is equal to the number of observations in the set of matrices of potential regulations.

After the identifying, for each identified bound transcription factor, of one or more potentially regulated genes, potentially regulator genes encoding the bound transcription factor are identified. This will allow to make links between regulator and regulated genes and not only transcription factor to regulated genes.

In examples, the identification of the one or more potentially regulator genes may be performed by using information known from the literature, e.g. regulator gene(s) coding a protein is(are) known.

In examples, the identification of the one or more potentially regulator genes may be performed by considering the transcription factor as the gene encoding it. Transcription factors are proteins, which means there is a gene that transcripts then translates to protein, and a protein often has the same name as the transcription factor.

From now, for each transcription factor, the gene encoding the transcription factor and all the genes regulates by the transcription factor are identified. The next step is to connect the regulator gene and the one or more regulated genes in order to obtain the matrix of potential regulations. Connecting means that a correspondence table between regulator gene, transcription factor and regulated gene is built.

In an example, each connection described for each time series of the observed biological process is equivalent to a connection described for one of the time series of the observed biological process. This ensures that no connection has been lost. The regulations map means therefore that a potential regulation was observed sometime during the observed biological process.

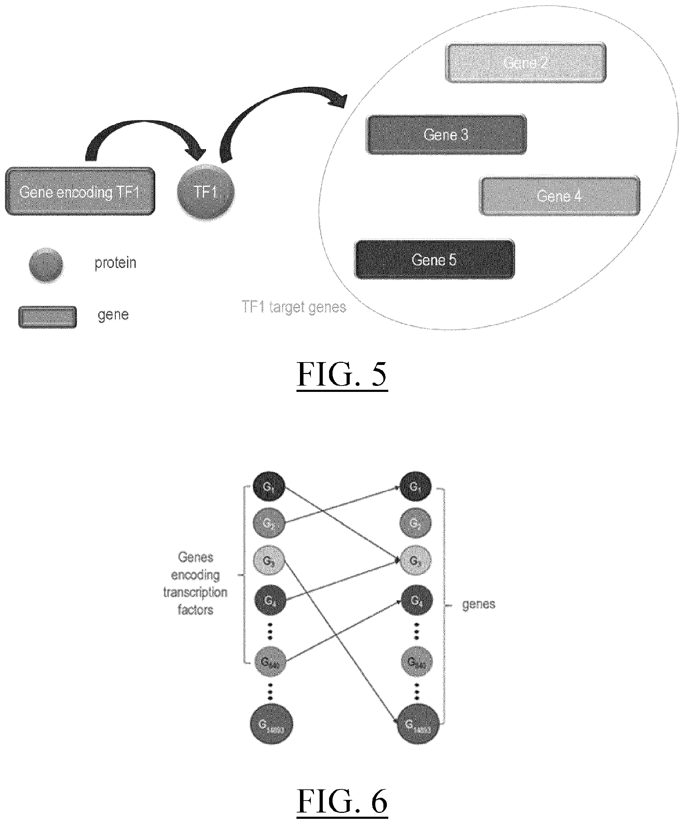

FIG. 5 is a schematical representation of the principle behind a matrix of potential regulations. For each gene encoding a transcription factor (noted TF1), target genes of TF1 are known.

The matrix is built from experimental data of the at least one time series of an observed biological process involving the genes of the set of genes of the sequence of the reference genome. For example, the observed regulations are observed in the set of experimental data used for building the matrix that is obtained (S10).

Referring back to FIG. 1, after S10, a neural network is provided (obtained) S20. The neural network has an input layer of nodes and an output layer of nodes. The input layer and the output layer have an equivalent node for representing each gene of the set of genes of the sequence of the reference genome. In other terms, the number of nodes of the input layer is equals to the number of nodes of the output layer. Each node of the input layer represents a regulator gene and each node of the output layer represents a regulated gene. A network structure with 2 layers is obtained. The network structure may be based on a any known neural network.

Then, at S30, connections are added to the neural network. A connection connects a node of the input layer with one or more nodes of the output layer. The added connections are extracted from the obtained matrix of potential regulations. Hence, for each couple of the regulation map, a link is created from the node in layer 1 representing the regulating gene towards the node in layer 2 representing the regulated gene. The resulting network thus reproduces all connections of the matrix of potential regulations. Therefore, the number of nodes in each layer of the neural network depends on the number of gene of the set of genes. For example, if the expression of 200 genes is measured, the neural network has a same number of 200 nodes in each layer without loss of information. If the expression of all genes is measured, the obtained neural network considers all genes. The binary information regulation/no regulation is transcribed as a link between two nodes (that do not belong to the same layer) of the network.

FIG. 6 is an illustration of a network structure obtained as a result of S20 and S30.

Next, the neural network (obtained after S30 has been carried out) is trained S40. The training step consists in varying weights and/or biases of the connections of the neural network. The training of the neural network may forces the weight of a connection to output values consistent with the (training) dataset. The neural network obtained after S30 may have connections with no weights or connection with weights that are equals, and where the (training) dataset provides training samples/examples each comprising time series of gene expression profiles of the observed biological process. The (training) dataset may comprise or consist of any final or intermediary result of the gene expression profiles of the observed biological process, or any post-processing of such observed biological process. For example, the (training) dataset may indicate (i.e., represent directly or indirectly) gene expression data such as a list of reference genes and a respective level of expression associated to each gene, for example expressed in Fragments Per Kilobase of transcript per Million mapped reads or FPKM. The (training) dataset may be in the form of textual data and/or numeric data (i.e., numbers and/or vectors of numbers) derived from such textual data. The (training) dataset may comprise or consist of one or more time series of the observed biological process; each time series is considered independent from the others.

Thus, the training of the neural network serves only to refine the structure of the network by adjusting the weights of the connections so that the output of the trained network converge to the outputs of the (training) dataset. Each connection of the trained the neural network is therefore weighted. The training of the sparse network is like a traditional neural network training except it is not fully connected.

At the time of finding the potentially regulated genes, it is not possible to know if the potential regulation is an up or down regulation. In a biological process, downregulation is the process by which a cell decreases the quantity of a cellular component, such as RNA or protein, in response to an external stimulus. The complementary process that involves increases of such components is called upregulation. The network training provides that information through the model study (the observed biological process). The weights of the links between nodes, considering that a positive weight means an up regulation, a negative weight a down regulation and a weight "close" to 0 not a regulation.

Next, S50, weighted connections of the trained neural network are removed if they have an insignificant weight value. Said otherwise, the potential regulations that were no regulation are removed. The removing may be performed after the training, or while the training is carried out. When performed after the training, an algorithm parses every connection of the learned network and removes connections with low weight. Here, low is defined as modification of gene expression in the range of experimental error. The selection of only significant links is performed in order to remove links that add noise to the model and make it imprecise.

In examples, the removing of a connection of the trained neural network is performed based on a predetermined weight value associated with connection starting from a given regulator gene. The predetermined value may be the same for one or more (all) connections starting from a given regulator gene.

In examples, the removing of a connections of the trained neural network may comprise performing an evaluation of the significance of each connection of the trained neural network. The evaluation of the significance may comprise obtaining a value of a threshold of insignificance representing a modification of an expression of the regulated gene in a range of an experimental error. "Experimental error" refers to a difference between a measured or estimated value for a quantity and its true value, and is inherent in all measurements. Experimental error may thus occur when for instance, a potential regulator gene is not involved in a time series of the observed biological process. If the weight value of a connection of the trained neural network is smaller than the threshold of insignificance computed after the training, then the connection to the regulated gene is removed. The experimental error for each regulator gene is known (e.g. a quality score is attributed to each gene expression, and the better is the quality score, the lower is the experimental error) and the impact of a weight on the prediction can be calculated. If the impact is less than the experimental error then the connection is removed.

After training, each weight on links (connections) between nodes are inferred, and if the weight of the link is indicative of a regulation that can be considered within the experimental error range, the link between the regulator and regulated genes is removed. At the end of this process S50, a new structure with fewer links is obtained (unless no weight value was indicative of a regulation considered within the experimental error range).

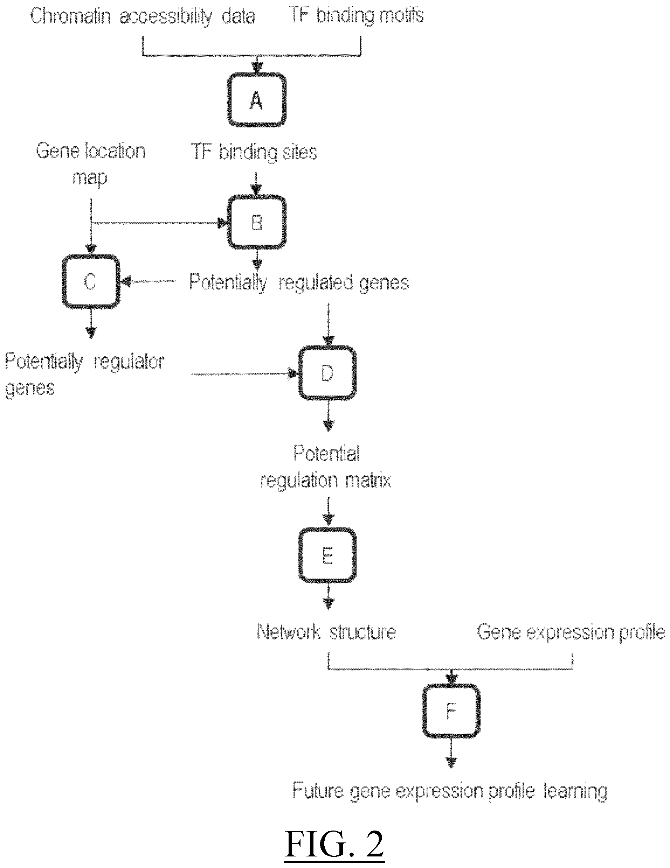

Referring now to FIG. 2, an example of implementation of the present method is discussed. This example of implementation combines several examples of the method that have been discussed and described hereinabove.

In this example of implementation, are provided as input data regarding chromatin accessibility, transcription factors binding motifs, a map of genes location, a set of gene expression profiles, the set being a time series of gene expression profile, and a sequence of reference genome.

The goal of the steps noted A, B and C is to extract information from chromatin accessibility data (for example DNAse-seq or ATAC-seq) to understand which genes may regulate which other genes in the studied biological process.

Step A finds transcription factor binding sites. As illustrated on FIG. 3, TFs bind to DNA to regulate transcription and in the end gene expression. For find transcription factors binds where, three external software tools are used for performing a peak calling, footprinting, and motif matching. This is performed as previously discussed and the output step A is a list of TF binding sites that may be seen as map of the genome involves in the observed biological process with the position and names of all potentially bound transcription factors.

Step B aims at understanding which transcription factor may regulate which gene. This is performed using the output of step and a gene location map as previously discussed. The output of B is a list of potentially regulated genes for each discovered TF binding site.

Step C finds the potentially regulator genes using in input the list of potentially regulated genes of step B and the gene location map. The output of C is a list of potentially regulator genes

At step D, information of step B and step C are combined, as illustrated by FIG. 5. The output of D is the so-called matrix of potential regulations.

At step E, the 2 layers network structure is created with, for each couple of the regulation map, a link from the node in layer 1 representing the regulating gene towards the node in layer 2 representing the regulated gene; FIG. 6 illustrates the result of step E.

Finally, at step F, the 2 layers network structure provided in output of step E is trained using a traditional neural network training. The selection of only significant links is performed in order to remove links that add noise to the model and make it imprecise.

Still in step F, the trained with fewer links is also used for to predicting future gene expression by providing in input of this trained model gene expressions to predict future gene expression. The trained model according to the examples of the invention can be used for inferring a gene expression profile. Input data is provided, including a gene expression for a time series of the observed biological process involving the genes of the set of genes of the sequence of the reference genome. The provided input is applied on the trained neural network to the input data to infer a future gene expression; this is performed as known in the art.

The trained model may be also trained again for further uses. In a first example, an experiment called "knock down" is often used by biologists to understand what happens if one or several genes are less (or more) expressed. This is a very expensive experiment and biologists chose attentively on which gene they perform the experiment. The present invention allows to do it in silico. To do so, the algorithm takes the gene expression profile for which one would like to know what implicates one or several genes modification and change the expression of the those genes. For example one could modify one gene expression to 130% of its original value (meaning an overexpression) and another to 30% of its original value (repression). The modified gene expression profile is given as input to the learned model and the prediction simulates the future gene expression profile given the modifications.

In a second example, an analysis of the weights on the connections between nodes allows to get an insight of the action of a gene on another one, e.g. does it up or down regulates it or nothing at all.

## Claims

1. A computer-implemented method for training a neural network for inferring a gene expression profile, the method comprising:
- obtaining (S10) a matrix of potential regulations between genes of a set of genes of a sequence of reference genome, the matrix of potential regulations describing connections between regulator genes and regulated genes, a regulator gene encoding at least one transcription factor regulating at least one regulated gene, a connection representing at least one observed regulation of the regulated gene by the regulator gene in at least one time series of an observed biological process involving the genes of the set of genes of the sequence of the reference genome;
- obtaining (S20) a neural network having an input layer of nodes and an output layer of nodes, the input layer and the output layer having an equivalent node for representing each gene of the set of genes of the sequence of the reference genome, each node of the input layer representing a regulator gene and each node of the output layer representing a regulated gene;
- adding (S30) connections to the neural network from the nodes of the input layer to the nodes of the output layer, the added connections being extracted from the obtained matrix of potential regulations;
- training (S40) the neural network by using a set of gene expression profiles of the observed biological process, each connection of the trained the neural network being weighted; and
- removing (S50) connections of the trained neural network having an insignificant weight value.

2. The computer-implemented method of claim 1, wherein the removing the connections of the trained neural network having an insignificant weight comprises performing for each connection of the trained neural network:
- obtaining a value of a threshold of insignificance representing a modification of an expression of the regulated gene in a range of an experimental error;
- removing the connection to the regulated gene if the weight value is smaller than the threshold of insignificance.

3. The computer-implemented method of any one of claims 1 to 2, wherein the obtained matrix of potential regulations between genes of a set of genes of a sequence of reference genome has been computed by:
- identifying, for each gene of the set of genes of the sequence of the reference genome, one or more transcription factor binding sites and the respective transcription factor(s) bound on the one or more transcription factor binding sites;
- for each identified bound transcription factor:
- identifying one or more potentially regulated genes;
- identifying a potentially regulator gene encoding the bound transcription factor; and
- connecting the regulator gene and the one or more regulated genes.

4. The computer-implemented method of claim 3, wherein the identifying one or more potentially regulated genes comprises:
- determining, from a gene location map of the genes of the set of genes of the sequence of the reference genome, if one or more genes are in the frame of a predetermined number of base pairs around the identified bound transcription factor; and
- identifying the one or more genes are in the frame of a predetermined number of base pairs around the identified bound transcription factor as potentially regulated genes.

5. The computer-implemented method of claim 4, wherein the predetermined number of base pairs is smaller than 15000, preferably smaller than 10000.

6. The computer-implemented method of any one of claims 3 to 5, wherein the identifying, for each gene of the set of genes of the sequence of the reference genome, one or more transcription factor binding sites comprises:
- performing a peak calling operation on chromatin accessibility data of the set of genes of the sequence of the reference genome, thereby identifying peaks;
- identifying one or more hollows for each identified peak, thereby obtaining footprints of a past presence of transcription factor on the chromatin accessibility data of the set of genes of the sequence of the reference genome;
- comparing the obtained footprints to motifs of known transcription factors; and
- identifying, as a result of the comparing, which transcriptions factor has been bound to each footprint.

7. The computer-implemented method of any one of claims 3 to 6, wherein the obtained matrix of potential regulations between genes of a set of genes of a sequence of reference genome has been computed by:
- obtaining a matrix of potential regulations for each time series of the observed biological process, thereby obtaining a set of matrices of potential regulations; and
- merging the matrix of potential regulations of the set of matrices of potential regulations.

8. The computer-implemented method of claim 7, wherein a connection described for each time series of the observed biological process is equivalent to a connection described for one of the time series of the observed biological process.

9. A computer-implemented method of use of the trained neural network for inferring a gene expression profile according to anyone of claims 1 to 8, comprising:
- providing input data including a gene expression for a time series of the observed biological process involving the genes of the set of genes of the sequence of the reference genome;
- applying the trained neural network to the input data to infer a future gene expression.

10. A computer-implemented method for obtaining a matrix of potential regulations according to anyone of claims 1 to 8.

11. A data structure comprising a trained neural network according to any one of claims 1 to 8, a dataset formed according to claim 10, and/or a computer program comprising instructions for performing the method of any one of claims 1 to 8, the method of claim 9, and/or the method of claim 10.

12. A computer readable storage medium having recorded thereon the data structure of claim 11.

13. A device comprising a data storage medium having recorded thereon the data structure of claim 11.
